# EUROPEAN PATENT SPECIFICATION

(11) **EP 4 440 621 B1**
(45) Date of publication and mention of the grant of the patent: **04.02.2026**
(21) Application number: 22829755.2
(22) Date of filing: 02.12.2022
(51) Int. Cl.: A61K 47/55, A61K 47/64, A61P 25/16, A61P 25/28

(54) **PEPTIDE-DRUG CONJUGATES FOR TREATMENT OF NEURODEGENERATIVE DISEASES**
PEPTID-ARZNEIMITTEL-KONJUGATE ZUR BEHANDLUNG VON NEURODEGENERATIVEN ERKRANKUNGEN
CONJUGUÉS PEPTIDE-MÉDICAMENT POUR LE TRAITEMENT DE MALADIES NEURODÉGÉNÉRATIVES

(30) Priority: 02.12.2021 EP 21212124
(43) Date of publication of application: 09.10.2024
(73) Proprietor: Københavns Universitet, 1165 Copenhagen K (DK)
(72) Inventor: CLEMMENSEN, Christoffer, 1165 Copenhagen K (DK); PETERSEN, Jonas Odgaard, 1165 Copenhagen K (DK); KLEIN, Anders Bue, 1165 Copenhagen K (DK)
(74) Representative: AWA Denmark A/S
(86) International application number: PCT/EP2022/084178
(87) International publication number: WO 2023/099723

(56) References cited:
- WO-A1-2011/094337
- WO-A1-2011/143208
- WO-A1-2021/245199
- US-A1- 2012 329 707
- HAMPSON A J ET AL: "DUAL EFFECTS OF ANANDAMIDE ON NMDA RECEPTOR-MEDIATED RESPONSES AND NEUROTRANSMISSION", JOURNAL OF NEUROCHEMISTRY, WILEY-BLACKWELL PUBLISHING LTD, GB, vol. 70, no. 2, 1 February 1998 (1998-02-01), pages 671 - 676, XP009058545, ISSN: 0022-3042
- ZHENG LIU ET AL: "Synthesis and biological evaluation of memantine nitrates as a potential treatment for neurodegenerative diseases", MEDCHEMCOMM, VOL. 8, N. 1, 20 October 2016 (2016-10-20), pages 135 - 147, XP055692256, Retrieved from the Internet <URL:http://xlink.rsc.org/?DOI=C6MD00509H> [retrieved on 20200506], DOI: 10.1039/C6MD00509H
- KEVIN K OGDEN ET AL: "New advances in NMDA receptor pharmacology", TRENDS IN PHARMACOLOGICAL SCIENCES, vol. 32, no. 12, 1 December 2011 (2011-12-01), pages 726 - 733, XP028119161, ISSN: 0165-6147, [retrieved on 20110817], DOI: 10.1016/J.TIPS.2011.08.003
- TRAYNELIS STEPHEN F. ET AL: "Glutamate Receptor Ion Channels: Structure, Regulation, and Function", PHARMACOLOGICAL REVIEWS, vol. 62, no. 3, 17 September 2010 (2010-09-17), US, pages 405 - 496, XP093029455, ISSN: 0031-6997, Retrieved from the Internet <URL:http://dx.doi.org/10.1124/pr.109.002451> DOI: 10.1124/pr.109.002451
- JALEWA JAISHREE ET AL: "A novel GLP-1/GIP dual receptor agonist protects from 6-OHDA lesion in a rat model of Parkinson's disease", NEUROPHARMACOLOGY, ELSEVIER, AMSTERDAM, NL, vol. 117, 20 February 2017 (2017-02-20), pages 238 - 248, XP029970772, ISSN: 0028-3908, DOI: 10.1016/J.NEUROPHARM.2017.02.013
- HÖLSCHER CHRISTIAN: "Brain insulin resistance: role in neurodegenerative disease and potential for targeting", vol. 29, no. 4, 16 March 2020 (2020-03-16), UK, pages 333 - 348, XP055914561, ISSN: 1354-3784, Retrieved from the Internet <URL:https://www.tandfonline.com/doi/pdf/10.1080/13543784.2020.1738383> DOI: 10.1080/13543784.2020.1738383

## Description

### Technical Field

The present invention relates to the field of therapeutic conjugates displaying agonism to receptors of the glucagon superfamily peptides and antagonism to the N-methyl-D-aspartate receptor for use in treatment and/or prevention of neurodegenerative diseases in a subject.

### Background Art

Neurodegeneration, also referred to as neurodegenerative diseases, characterize complex and serious medical conditions, which principally affect the neurons in the human brain. Neurodegeneration can be found in the brain at many different levels of neuronal circuitry, ranging from molecular to systemic. Because there is no known way to reverse the progressive degeneration of neurons, neurodegenerative diseases are considered to be incurable. Neurodegenerative diseases such as dementia, Alzheimer's disease, Parkinson's disease, Huntington's disease, all occur as a result of neurodegenerative processes.

Alzheimer's disease is the most common neurodegenerative disease and is characterized by loss of neurons and synapses in the cerebral cortex and certain subcortical regions. Most of the Alzheimer's Disease drug development programs have been discouraging, presenting poor efficacy, founded on symptomatic relief and with little or no impact on disease reversal. Currently, two major drug classes are approved and used for treatment of Alzheimer's Disease, namely acetylcholine esterase inhibitors and N-methyl-D-aspartate (NMDA) receptor antagonists.

NMDA receptors (NMDARs) are a subclass of glutamate receptors that requires both binding of glutamate and postsynaptic depolarization for their activation, and that mediates Ca²⁺ entry when they are activated. NMDAR dysfunction, arising e.g. from altered receptor-channel activity, subunit expression, trafficking, or localization, may contribute to a variety of neurological and psy-chiatric conditions and, therefore, the involvement of NMDARs in different neurological diseases has been subject to increased attention over the years.

NMDAR antagonists, such as e.g. memantine under the Tradename Ebixa^{®}, are currently used for treatment of Alzheimer's disease and are available as generic medication and used for treating millions of people worldwide. The non-competitive NMDAR antagonist memantine belongs to the group of open-channel blockers of the NMDA receptor.

US 2004 102525 A1 describes methods of treatment of acute, chronic and prophylactic treatment of neurologic and neurodegenerative diseases, including Alzheimer's disease, using memantine.

The glucagon superfamily comprises the peptides glucagon, secretin, vasoactive inhibitory peptide (VIP), gastric inhibitory peptide (GIP), growth hormone-releasing factor (GHRF), as well as Glucagon-like peptide 1 (GLP-1) and GLP-2. These peptides exert diverse actions on nutrient intake, gastrointestinal motility, islet hormone secretion, cell proliferation and apoptosis, nutrient absorption, and nutrient assimilation. Recent studies have further shown that these peptides may also have a neuroprotective role in the brain for which reason their relevance in treating neurodegenerative diseases is now gaining increased attention.

GIP is derived from a 153 amino acid long proprotein encoded by the GIP gene and circulates as a biologically active 42 amino acid long peptide. The GIP receptor (GIPR) is expressed in various tissues and has been found in several brain regions with high levels of expression in the olfactory bulb and in the large pyramidal neurons in the hippocampus and cerebral cortex. Activation of GIPR leads to proliferation of neuronal progenitor cells and therefore may contribute to neurogenesis.

GLP-1 is a 30 or 31 amino acid long peptide hormone derived from the tissue-specific posttranslational processing of the proglucagon peptide. GLP-1 receptor agonists are widely acknowledged for the anti-diabetes and anti-obesity effects. More recently, it has been demonstrated that long-acting GLP-1 receptor (GLP-1R) agonists have neuroprotective potential, and some companies are now scrutinizing the utility of GLP-1R agonists for treatment of Alzheimer's disease in clinical phase III trials.

WO 2021/089678 A1 describes the use of GLP-1R agonists, in particular semaglutide, to treat or reduce the risk of developing dementia in subjects with metabolic syndrome.

WO 2011 143208 A1 discloses glucagon superfamily peptide agonists conjugated to G protein-coupled receptor (GPCR) ligands for use in treating metabolic syndrome, diabetes, obesity, liver steatosis, and a neurodegenerative disease. The GPCR ligand may be the eicosanoid anandamide. Hampson A. J. et al., Journal of neurochemistry, 70, 2: 671-676 (1998) describes the role of the eicosanoid anandamide on modulation of NMDA receptor activity.

WO 2011 094337 A1 discloses conjugates of peptide combinations comprising a GIP agonist peptide and a glucagon antagonist peptide for treating metabolic disorder, such as diabetes and obesity. The peptides described in WO 2011 094337 A1 may further be co-administered with an agent for treatment of a neurodegenerative disease.

US 2012 329707 A1 discloses conjugates of glucagon/GLP-1 receptor co-agonists and their use in treating metabolic syndrome, diabetes, obesity, liver steatosis, and neurodegenerative disease.

Zheng Liu et al., Med. Chem. Commun. 8, 1:135-147 (2016) discloses biological evaluation of newly synthesized memantine nitrates as a potential treatment for neurodegenerative diseases, including Alzheimer's disease.

WO 2021 245199 A1 discloses conjugates of GLP1 receptor agonist and NMDA receptor antagonist and the use of these conjugates in treatment of obesity and obesity-related disorders.

With the aging of the population, the incidence of neurodegeneration is increasing dramatically in the absence of either effective therapeutic interventions or a clear understanding of the discrete pathophysiology of neurodegenerative disease states.

Thus, there remains an unmet need for improved medicinal treatment of neurodegenerative diseases with greater efficacy and higher safety (low toxicological effect).

### Summary of the invention

On this background it is an object of the present invention to provide improved therapies for use in the treatment and/or prevention of neurodegenerative diseases that entail fewer side effects and comprise administering a therapeutic agent that is better tolerated by the subject.

Accordingly, a first aspect of the present invention relates to a conjugated molecule for use in treatment and/or prevention of a neurodegenerative disease in a subject in need thereof, the conjugated molecule comprising a peptide and a N-methyl-D-aspartate receptor (NMDAR) antagonist, wherein the peptide is according to SEQ ID NO:1, SEQ ID NO:5, SEQ ID NO:6, SEQ ID NO:7 or SEQ ID NO:8, , the peptide being linked to the NMDAR antagonist either directly or through a chemical linker, wherein the NMDAR antagonist is selected from memantine, neramexane, and dizocilpine.

The inventors have surprisingly found that the conjugate of the present invention is effective in the treatment or prevention of a neurodegenerative diseases, in particular in the treatment and prevention of Alzheimer's disease, in a subject. It is postulated, that this novel medicinal strategy harnesses the benefits of both molecular entities, that is glucagon superfamily peptide receptor agonism and NMDA receptor antagonism, in a single molecule. The inventors have demonstrated that the combination of the two neuroprotective modalities in a conjugated molecule elicits improved neuroprotective effects in *in vitro* and *in vivo* models of NMDA induced cytotoxicity and in the most commonly used transgenic AD mouse model (5xFAD), respectively. To the inventors' knowledge, none of the existing treatment strategies, pre-clinical as well as clinical, for neurodegenerative diseases combines NMDA receptor antagonism with the specific members of the glucagon superfamily peptide receptor agonism.

Without being bound by any particular theory, the inventors speculate that the neuroprotective effect of the conjugated molecule according to the invention is achieved by the NMDAR antagonist accumulating at and/or close to the sites of glucagon superfamily peptide receptors in the body due to the affinity of the peptide towards glucagon superfamily peptide receptors.

A peptide will have an amino terminus and a carboxyl terminus. In the context of the invention, the amino terminus and the carboxyl terminus of the peptide may also be referred to as the N-terminus and the C-terminus, respectively, and corresponding derived forms.

The peptide according to the present invention may consist of amino acids encoded by the genetic code or it may contain amino acids encoded by the genetic code and natural amino acids, which are not encoded by the genetic code, such as D-alanine (dAla). Further, the peptide according to the invention may incorporate synthetic amino acids such as D-alanine, or a-aminoisobutyric acid (Aib), d-Serine (dSer), N-methyl-serine.

In an embodiment, the amino acid on position 2 (counted from the N-terminal) of the peptide is D-alanine, D-serine, alpha-aminoisobutyric acid, N-methyl-serine, glycine, or valine.

The peptide according to the invention may also have one or more modifications to stabilise its secondary structure, such as cyclisation between a glutamic acid on position 15 and a lysine on position 20 of the peptide, the amino acid positions being counted from the N-terminal of the peptide.

The peptide may be obtained from any source or the peptide may be produced as desired. For example, the peptide may be isolated from a tissue, or the peptide may be produced recombinantly or synthesized by methods that are well known to the person skilled in the art.

The peptide of the conjugate according to the present invention belongs to the glucagon superfamily of peptides. The glucagon superfamily is a group of peptides related in structure in their N-terminal and C-terminal regions (see, for example, Sherwood et al., Endocrine Reviews 21: 619-670 (2000)). Members of this group include all glucagon related peptides, as well as Growth Hormone Releasing Hormone, vasoactive intestinal peptide, pituitary adenylate cyclase-activating polypeptide 27, Secretin, modified GLP-1 (SEQ ID NO:1), unmodified GLP-1 (SEQ ID NO:2), Exendin-4 (SEQ ID NO:3), Gastric inhibitory peptide (GIP) (SEQ ID NO:4), GLP-1/GIP co-agonists (SEQ ID NO:5 and SEQ ID NO:6), GLP-1/GIP/Glucagon (Gcg) tri-agonist (SEQ ID NO: 7), and analogues, derivatives or conjugates with up to 1, 2, 3, 4, 5, 6, 7, 8, 9 or 10 amino acid modifications relative to the native peptide.

In an embodiment, the peptide according to the invention is an incretin hormone. Peptide hormones of the incretin family include, but are not limited, to GLP-1, GIP, combinations and/or analogues thereof.

The peptide according to the invention retains the ability to interact as an agonist with receptors of the glucagon receptor superfamily. As used herein, an "agonist" is a molecule, such as e.g. a hormone or a drug, that attaches or binds to a cellular receptor to produce an effect on the cell. The binding of the agonist to the given receptor elicits a biological response.

In an embodiment, the peptide is a GLP-1 peptide, a GIP peptide, a combination thereof, and/or any derivatives or analogues thereof which interacts with the GLP-1 and/or GIP receptor as agonist.

In specific embodiments, the peptide of the conjugated molecule has the amino acid sequence of SEQ ID NO:5. In other specific embodiments, the peptide of the conjugated molecule has the amino acid sequence of SEQ ID NO:6 or SEQ ID NO:7.

Also contemplated are peptides with co-agonist and tri-agonist activity. Peptides with co- or tri-agonist activity display the ability to bind to different receptors, two or more, of the glucagon receptor superfamily. In one embodiment, the co-agonist is a GLP-1/GIP receptor co-agonist. In an embodiment, the GLP-1/GIP receptor co-agonist is according to SEQ ID NO: 5. In an embodiment, the GLP-1/GIP receptor co-agonist is according to SEQ ID NO: 6. Also contemplated are peptides with co-agonist activity to the GLP-1/Gcg receptors or GIP/Gcg receptors. In another embodiment, the tri-agonist is a GLP-1/GIP/Gcg receptor tri-agonist. In an embodiment, the tri-agonist is according to SEQ ID NO:7.

In an embodiment, the peptide of the conjugated molecule according to the invention has at least 85% amino acid sequence identity to SEQ ID NO:1. For example, the peptide may have at least about 90% identity to SEQ ID NO:1. In a further embodiment, the peptide has at least 95% amino acid sequence identity to SEQ ID NO:1.

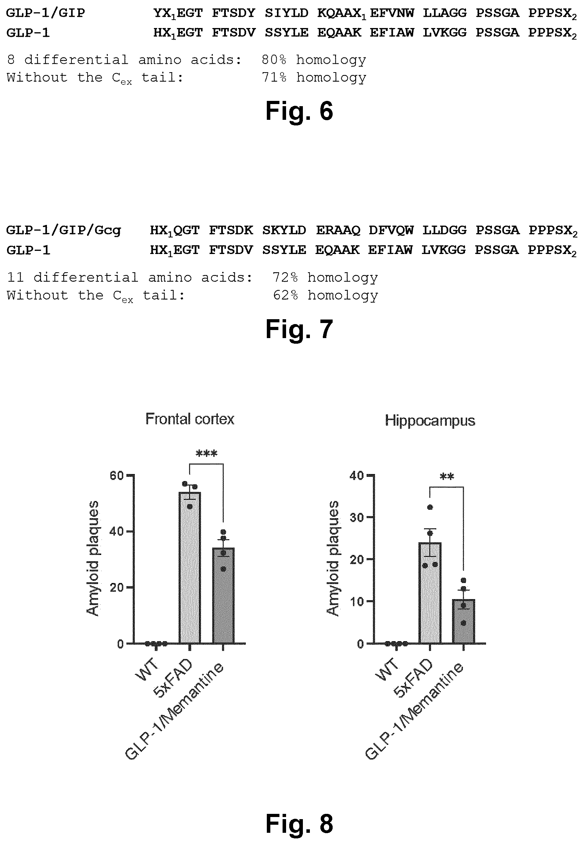

In an embodiment, the peptide is according to the conserved amino acid sequence according to SEQ ID NO: 8. In the context of the present invention, a peptide according to SEQ ID NO: 8 includes, in addition to its conserved amino acids, any natural or synthetic amino acid at the amino acid positions 1, 10, 12, 15, 16, 23, 24, 27, 28, 30, 31 counted from the N-terminal of the peptide according to SEQ ID NO. 8. Such a peptide is exemplified by the GLP-1/GIP co-agonist peptide according to the amino acid sequence of SEQ ID NO:5, see Fig. 6.

In an embodiment, the peptide according to the invention has the amino acid sequence according to SEQ ID NO:1. Such a peptide may have a significantly greater GLP-1 activity at the GLP-1 receptor compared to native GLP-1 (unmodified GLP-1, SEQ ID NO:2) at the GLP-1 receptor. Also contemplated is a GLP-1 receptor peptide having at least 85% amino acid sequence identity to SEQ ID NO:1, but without the exendin-4 extension at the C-terminal of the peptide. In the context of the present invention, the exendin-4 extension has the amino acid sequence GPSSGAPPPS (SEQ ID NO:9).

The peptide according to the invention may, if conjugated to an NMDAR antagonist, accumulate at a greater rate at the site of the glucagon superfamily peptide receptors, which in turn may lead to a greater efficacy of the NMDAR antagonist. An example of a peptide having at least 80% amino acid sequence identity to SEQ ID NO: 1 is shown in Fig. 6. An peptide of the conjugate having at least 70% amino acid sequence identity to SEQ ID NO: 1 is shown in Fig. 7. The alignment between GLP-1/GIP (peptide according to SEQ ID NO:5) and GLP-1 (peptide according to SEQ ID NO:1), as shown in Fig. 6, is further illustrated below:

| | |
|---|---|
| GLP-1/GIP | YX₁EGT FTSDY SIYLD KQAAX₁ EFVNK LLAGG PSSGA PPPSX₂ |
| GLP-1 | HX₁EGT FTSDV SSYLE EQAAK EFIAW LVKGG PSSGA PPPSX₂ |

The peptide of the conjugated molecule will have a length sufficient for the peptide (in its free form), to display at least 0.1 % activity of native glucagon superfamily peptide at the glucagon superfamily peptide receptor. In general, this can be observed for peptides comprising at least 10 amino acids, but the activity may not be displayed when the peptide comprises more than 60 amino acids. Thus, in an embodiment, the peptide has a length in the range of 10 to 60 amino acids, e.g. 20 to 50 amino acids. Amino acid sequences of the present invention that are identical to other peptides sequences to a certain percentage should comprise enough of the amino acid sequence of a peptide, e.g. at least 10 amino acids, to afford putative identification of that peptide, either by manual evaluation of the sequence by one skilled in the art, or by computer-automated sequence comparison and identification using algorithms such as BLAST (Basic Local Alignment Search Tool) (for a review see Altschul, et al., Meth Enzymol. 266: 460,1996; and Altschul, et al., Nature Genet. 6: 119, 1994).

In the context of the present invention, peptide may differ in %-identity by having substitutions, insertions of natural or synthetic amino acids and/or having amino acid deletions.

In an embodiment, the peptide is modified by acetylation, fatty acid conjugation, diacid conjugation, albumin conjugation, small-molecule albumin binders, and/or PEG conjugation. Also contemplated are peptides modified by linking to carrier proteins, such as antibodies. The modifications are preferably at position 16, 17, 20, 21, 24, 29, 40 of the peptide (counted from the N-terminal), within a C-terminal region, or at the C-terminal amino acid. The conjugation may be made by any suitable linker, such as by disulfide, maleimide, alpha-ketone, or click-chemistry based conjugation. The skilled person knows how to prepare such conjugates. Preferably, PEG molecules may be larger than 1 kDa and fatty acids and diacids may contain more than 12 carbon atoms. It is generally preferred to add a spacer between the modification (PEG/fatty acid/diacid) and the peptide, the linker preferably being a gamma-Glu linker, a short PEG chain.

The NMDA receptor antagonist will bind to the NMDA receptor, and the NMDAR antagonist may be described as having a dissociation constant K_{d} with a specified NMDA receptor, e.g. in the free form of the NMDAR antagonist. NMDAR antagonists generally have dissociation constants in the nanomolar range, for instance the dissociation constant of MK801 (dizocilpine) with NMDA receptors of different species are K_{d} = 6.3 nM in brain membranes of rats, K_{d} = 10 nM in brain homogenates of mice, and K_{d} 1.3 nM in pig brains. Determination of dissociation constants is well-known to the skilled person. In an embodiment, the NMDAR antagonist in its free form has a dissociation constant K_{d} with an NMDA receptor in the range of 0.5 nM to 1000 nM, e.g. in the range of 0.5 nM to 100 nM. The NMDA receptor may for example be a human NMDA receptor, e.g. the NMDAR antagonist has a K_{d} with human NMDA receptor in the range of 0.5 nM to 100 nM. In the context of the present invention, the NMDA receptor antagonist in its free form refers to the antagonist not being bound, especially chemically linked, to any chemical group and thus being in its native, unmodified form. A person skilled in the art will appreciate that only minor species variation between NMDA receptors is to be expected. It follows that a K_{d} value measured for rodents, such as mice or rats, or measured for higher mammals, such a pigs, would be expected to be similar to a K_{d} value measured for human NDMA receptors or other relevant animal or mammalian NMDA receptors.

An NMDAR antagonist is used with the conjugate. As used herein, an "antagonist" is a type of receptor ligand or drug that blocks or dampens a biological response by binding to and blocking a receptor rather than activating it like an agonist. Channel blockers are antagonists for the respective ion channels. The NMDAR antagonist according to the invention is selected from the group of open-channel blockers of the NMDA receptor. Open-channel blockers of the NMDA receptor may function by their noncompetitive binding to a site inside the channels pore that is located near the extracellular entrance. Their binding requires the channel to be open and there presence thus physically blocks ionic flow through the channel.

Examples of open-channel blockers of the NMDA receptor are for example disclosed in Traynelis et al., Pharmacological reviews, 62, 3: 405-496 (2010) and in Ogden and Traynelis, Trends in pharmacological sciences, 32, 12: 726-733 (2011), and include, but are not limited to dizocilpine, memantine, ketamine, norketamine, hydroxynorketamine, amantadine, dextromethorphan, levomethorphan, dextrorphan, levorphanol, phencyclidine (PCP), 1-phenylcyclohexylamine (PCA), CNS-1102 (Aptiganel), remacimide, pentamidine, 9-aminoacridine, 1,2,3,4-tetrahydro-9-aminoacridine (tacrine), 7-methoxytacrine, lanicemine.

In the context of the present invention, the NMDAR antagonist is a small molecule, e.g up to 900 kDa. In an embodiment, the NMDAR antagonist is selected from dizocilpine, also known as MK801, memantine, and neramexane Neramexane is a non-limiting example of a compound related to memantine.

The peptide of the invention and the NMDAR antagonist are linked. In the context of the present invention, the conjugated molecule may also be referred to as a peptide-drug-conjugate (PDC). The peptide and the NMDAR antagonist may be linked or bonded directly to each other. For example, the NMDAR antagonist may be linked through an amide bond, the amide bond being from the amino group on a NMDAR antagonist to a carboxylic acid group on the peptide. Such an amide bond may be made to any residue on the peptide having a carboxylic acid group such as a glutamic acid residue, an aspartic acid residue, a synthetic residue with a carboxylic acid group, or the carboxylic acid of the C-terminal. In an embodiment the NMDAR is memantine. For example, when the NMDAR antagonist is memantine, the amine of memantine may be bound to a carboxylic acid of an amino acid residue of the peptide.

In an embodiment, the conjugated molecule is for use in treatment and/or prevention of a neurodegenerative disease in a subject in need thereof, the conjugated molecule comprising a peptide having the amino acid sequence according to SEQ ID NO:1, wherein the amino acid on position 2 (counted from the N-terminal) of the peptide is alpha-aminoisobutyric acid, and the NMDAR antagonist is memantine, the peptide being linked to the NMDAR antagonist either directly or through a chemical linker. In another embodiment, the peptide is according to SEQ ID NO:1, NMDAR is memantine and the chemical linker is selected from linkers comprising a disulfide group.

In the context of the present invention, being directly linked means that the peptide has a covalent bond with the NMDAR antagonist, e.g. there are no additional chemical groups between the two molecules, such as a linker group. The peptide and the NMDAR antagonist may also be linked through a chemical linker. Any chemical linker may be used. However, it is generally preferred that the chemical linker has a length of up to 30 atoms. A longer chain may have the advantage of distancing the NMDAR antagonist from the peptide, such that the NMDAR antagonist is of no or little steric hindrance to the peptide, when the peptide interacts with a glucagon superfamily peptide receptor. No or low steric hindrance of the peptide affords a greater affinity towards the glucagon superfamily peptide receptor. A conjugate with a greater affinity towards the glucagon superfamily peptide receptor is likely to have a greater accumulation at the site of glucagon superfamily peptide receptors. The chemical linkers are preferably cleavable linkers, such as acid-cleavable linkers, enzyme-cleavable linkers, peptide-cleavable linkers, or disulfide linkers, which are generally well-known in the art for their use in peptide-drug conjugates. Examples of such cleavable linkers are compounds comprising glucuronide, beta-galactoside, disulfide, hydrazone and/or which compounds are cleavable by galactosidases, glucuronidases, pyrophospatases, phosphatases, arylsulfatases, proteases, or esterases. For instance, a linker may comprise a peptide cleavable by cathepsin, such as GFLG. The linker may further comprise a 4-aminobenzoic acid (PAB), that may be linked to the amino group of the NMDAR antagonist through an amide or carbamate bond. The linkers preferably release the NMDAR antagonist in its free form (i.e. native form), which may be achieved by many different linker chemistries such as the disulfide linkers disclosed herein. These linker chemistries and additional linker chemistries are well-known by the skilled person.

The NMDAR antagonist may be linked at the C-terminal region of the peptide. In the context of the invention, the C-terminal region may be up to 50% of the amino acids counted from the C-terminus, such as up to 40%, 30%, 25%, 20%, or 10% of the amino acids counted from the C-terminus. For instance, the C-terminal region of SEQ ID NO:1 may be amino acids 21 to 40, 26 to 40, or 31 to 40 (numbers counted from N-terminal). Thus, the NMDAR antagonist, e.g. memantine or dizocilpine, may be bonded, either directly or via a linker, to any one of the 10 amino acids counted from the C-terminus. For example, the NMDAR antagonist, e.g. memantine, may be linked directly to an amino acid within 5 amino acids from the C-terminus. Thereby the NMDAR antagonist produces little or no steric hindrance at the N-terminal of the peptide. As the N-terminal is involved in binding to the glucagon superfamily peptide receptor, no or low steric hindrance of the N-terminal may afford a greater affinity towards the glucagon superfamily peptide receptor. It is also contemplated that more than one NMDAR antagonist may be bonded to the same peptide molecule.

In one embodiment, the NMDAR antagonist is linked to the peptide via a chemical linker comprising a disulfide group. A disulfide group allows that the NMDAR antagonist is released from the peptide when chemically reduced. A chemical linker comprising a disulfide group, also known as a disulfide linker, ensures that the peptide and the NMDAR antagonist of the conjugate remain conjugated for an extended period during systemic circulation. The disulfide group of the disulfide linker may be reduced in a reducing environment, such as an intracellular environment, resulting in the conjugate being cleaved such that the peptide part of the conjugate is separated from the NMDAR antagonist part of the conjugate. The reduction may be through disulfide exchange with e.g. a thiol, such as glutathione or reductases such as intracellular protein disulfide-isomerase enzymes.

The chemical linker may be chosen from chemical linkers known in the art with the general formula R'-S-S-R", in which the R' and R" groups may be identical or different from each other. Advantageously, the conjugate may accumulate at and/or close to the sites of glucagon superfamily peptide receptors in the body due to the affinity of the peptide towards glucagon superfamily peptide receptors, and the NMDAR antagonist may be released at the sites and/or close to the sites of the glucagon superfamily peptide receptors. When free from peptide part of the conjugate, the NMDAR antagonist may have a suitably effect as site-specific NMDAR binding. It is speculated by the inventors that the conjugate may be cleaved in the extracellular environment immediately adjacent to cells harbouring glucagon superfamily peptide receptors, or that the conjugate may be internalized by the cells harbouring glucagon superfamily peptide receptors and cleaved in the reducing environment of the cells.

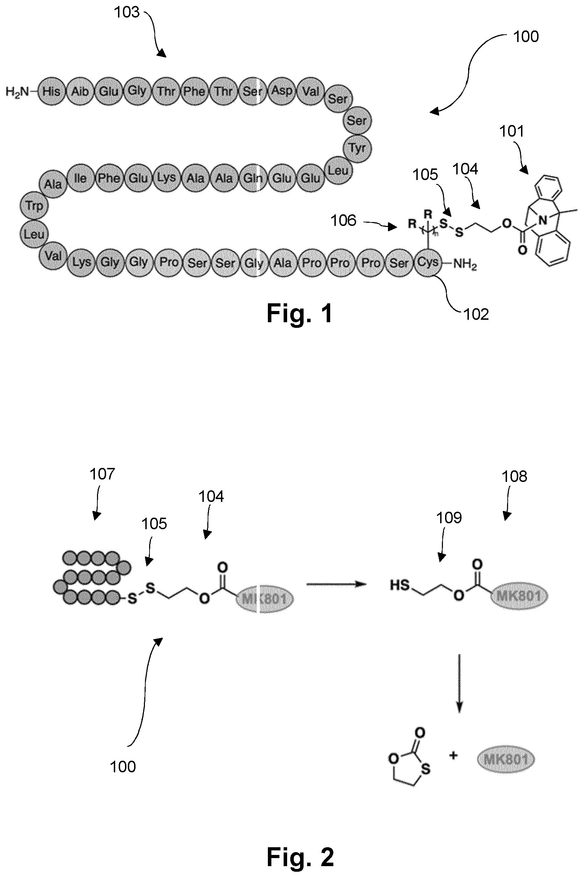

In an embodiment, the conjugated molecule is conjugated via a chemical linker, wherein the chemical linker has the formula R₁-R₃-S-S-R₄-R₅-O-CO-R₂, wherein R₁ is the peptide, R₂ the NMDAR antagonist, R₃ is optional and when present is selected from C(CH₃)₂, CH₂-CH₂, or CH₂, bonded to a side chain of the peptide or to a carbon atom of the backbone chain of the peptide, R₄ is (CH₂)ₙ or C₆H₄, Rs is optional and when present is selected from C(CH₃)₂, CH₂-CH₂, or CH₂, and n is 1, 2, or 3. When the chemical linker is reduced, the liberated NMDAR antagonist part of the conjugate undergoes intramolecular cyclisation which leads to the release of the NMDAR antagonist into its free form, see Fig. 2.

In one embodiment, the chemical linker has the formula R₁-R₃-S-S-(CH₂)ₙ-O-CO-R₂, wherein R₁ is the peptide, R₂ the NMDAR antagonist, R₃ is optional and when present is selected from C(CH₃)₂, CH₂-CH₂, or CH₂, bonded to a side chain of the peptide or to a carbon atom of the backbone chain of the peptide, and n is 1, 2, or 3.

In one embodiment, the chemical linker has the formula R₁-R₄-R₃-S-S-(CH₂)ₙ-O-CO-R₂, wherein R₁ is the peptide, R₂ the NMDAR antagonist, R₃ is optional and when present is selected from CH(CH₃)₂, CH₂-CH₂, or CH₂, bonded to a side chain of the peptide or to a carbon atom of the backbone chain of the peptide, R₄ is optional and when present is selected from CH(CH₃)₂, CH₂-CH₂, or CH₂, bonded to a side chain of the peptide or to a carbon atom of the backbone chain of the peptide and n is 1, 2, or 3.

In an embodiment, the second radical bond is to the backbone of the peptide of the invention. In another embodiment, the second radical bond is to a side chain of the peptide of the invention.

In the context of the present invention, when R₁ is bonded to the backbone chain of the peptide, C(CH₃)₂ (L-penicillamine) may be referred to as Pen, CH₂-CH₂ (L-homocysteine) may be referred to as hCys, and CH₂ (L-Cysteine) may be referred to as Cys, see Fig. 1.

As used herein, the first and the second radical bond is used to state the presence of at least two free bonds in the chemical linkers disclosed herein.

The present invention facilitates the design and synthesis of a library of conjugated molecules comprising a peptide and an NMDAR antagonist appended via chemical linkers. Fig. 1 shows how such conjugated molecules, may be designed. As shown in Fig. 1, the conjugate may be prepared by chemically bonding an NMDAR antagonist (MK801 in Fig. 1) to a peptide. The skilled person will appreciate that a vast number of different chemical linkers may be prepared by the methods disclosed herein and by other methods reported in literature, and these chemical linkers may be used to append peptides and NMDAR antagonists according to the methods disclosed herein and as reported elsewhere in the known art.

The inventors have found that the peptide of the present invention may serve as a targeting agent, allowing for site-selective delivery of otherwise nonspecific small-molecules, such as NMDAR antagonists, to regions of the brain.

The conjugated molecule disclosed herein provides selectivity and also up-concentrates drug action in the targeted region. This targeting enabled by the conjugated molecule allows for an improved therapeutic index, i.e. a lower minimum effective concentration. Tissue-selective targeting of NMDARs may be used for the targeted treatment of neurological diseases.

The inventors have demonstrated a surprising synergistic effect of the conjugates of the invention on neurodegeneration and this is significantly greater in comparison to the effect obtained with the administration of peptide alone, see Figs. 3 and 4. Furthermore, the prolonged administration of the conjugated molecule according to the invention seems not to affect the body weight of the mice used for the studies, see Fig. 5.

The data disclosed in the present invention have been obtained in studies of mice, but the conclusions are equally relevant for humans, since the major hormonal pathways governing energy metabolism are similar between mice and humans at they display comparable receptor expression profiles.

In one embodiment, the neurodegenerative disease which is treated with the conjugate molecule of the present invention, is selected from dementia, mild cognitive impairment, Alzheimer's disease, Parkinson's disease, stroke, traumatic brain injury, Huntington's disease, schizophrenia and depression. In one embodiment, the conjugated molecule according to the invention is for use in the treatment and/or prevention of Alzheimer's disease. There are five stages associated with Alzheimer's disease: preclinical Alzheimer's disease, mild cognitive impairment due to Alzheimer's disease, mild dementia due to Alzheimer's disease, moderate dementia due to Alzheimer's disease and severe dementia due to Alzheimer's disease. It is postulated that the conjugate molecule according to the invention may be used to treat Alzheimer's disease at any stage in the disease. Similarly, the conjugated molecule of the present invention may be used to treat other neurodegenerative diseases independent of the stage or progression of the disease.

In one embodiment, the conjugated molecule is administered in the form of a pharmaceutical composition, wherein the pharmaceutical composition comprises the conjugated molecule or a pharmaceutically acceptable salt thereof, and a pharmaceutically acceptable carrier. Any embodiment of the conjugated molecule may be used in the pharmaceutical composition.

The pharmaceutical formulations may be prepared by conventional techniques. Briefly, pharmaceutically acceptable carriers can be either solid or liquid. Solid form preparations include powders, tablets, pills, capsules, cachets, suppositories, and dispersible granules. A solid carrier can be one or more excipients which may also act as diluents, solubilisers, lubricants, suspending agents, binders, preservatives, wetting agents, tablet disintegrating agents or an encapsulating material.

The conjugate comprised in the pharmaceutical formulation may be in powder form, obtained by aseptic isolation of sterile solid or by lyophilisation from solution for constitution before use with a suitable vehicle, e.g., sterile, pyrogen-free water.

In one embodiment, the pharmaceutical composition is suited for subcutaneous administration, intramuscular administration, intraperitoneal administration, intravenous administration or for oral administration. Accordingly, the compositions of the present invention may be provided in unit dose form in ampoules, pre-filled syringes, small volume infusion or in multi-dose containers, optionally with an added preservative. The compositions may take such forms as suspensions, solutions or emulsions in oily or aqueous vehicles.

In an embodiment, the conjugated molecule or the pharmaceutical composition thereof for use in the treatment and/or prevention of a neurodegenerative disease is administered at least once a week for 12 month or more. In another embodiment, the conjugated molecule or the pharmaceutical composition thereof is administered 2-3 times a week for 12 months or more. In another embodiment, the conjugated molecule or the pharmaceutical composition thereof is administered to the subject once daily for 12 months or more.

The conjugated molecule according to the invention may be administered as a chronic treatment in which the conjugated molecule or a pharmaceutical composition comprising the conjugated molecule is administered for at least 12 months, such as for at least 16 months or at least 18 months. It is understood that chronic treatment with the conjugated molecule or the pharmaceutical composition thereof according to the invention may entail treatment carried out over a span of several years. In an embodiment, the conjugated molecule or the pharmaceutical composition thereof according to the invention is administered once daily or once a week for 1 year, 2 years, 5 years, 10 years, or more. In an embodiment, the conjugated molecule or the pharmaceutical composition thereof for treatment according to the invention is administered or 2-3 times a week for 5 years or more.

In the above, the invention has mainly been described with reference to a few embodiments.

Other aspects and advantageous features of the present invention are described in detail and illustrated by working examples below.

Generally, all terms used herein are to be interpreted according to their ordinary meaning in the technical field, and applicable to all aspects and embodiments of the invention, unless explicitly defined or stated otherwise. All references to "a/an/the [conjugate, molecule, linker, peptide, etc.]" are to be interpreted openly as referring to at least one instance of said conjugate, agent, molecule, linker, peptide, etc., unless explicitly stated otherwise.

In the context of the present invention, the term "peptide" means a compound composed of stretch of 10 to 60 amino acids connected by peptide bonds and belonging to the glucagon-superfamily of peptides. Such peptides include, but are not limited to, the incretin hormones glucagon-like peptide 1 (GLP-1), gastric inhibitory peptide (GIP) and glucagon (GCG). The peptide of the invention is an agonist of the glucagon superfamily peptide receptors and may also be considered to be a neuroprotective peptide and to function as an active delivery agent of the conjugated molecule of the present invention to sites in the brain, including the hypothalamus.

The term "derivative" or "analogue" as used herein in relation to a peptide or an amino acid means a chemically modified peptide or amino acid, wherein at least one substituent is not present in the unmodified peptide or amino acid or analogues thereof, i.e. a peptide or an amino acid which has been covalently modified. Typical modifications are amides, carbohydrates, alkyl groups, acyl groups, esters and the like.

In the context of the present invention, the term "percentage identity" or "% identity" means % of identical amino acids between two compared peptides, in particular using the BLAST algorithm.

The term "NMDAR antagonist" as used herein means a compound which is an antagonist of the NMDA receptor (NMDAR). Examples of NMDAR antagonist which belongs to the group of open channel blockers of the NMDA receptor include, but are not limited to, 3,5-dimethyladamantan-1-amine (memantine), 3,5-dimethyladamantan-1-amine;hydrochloride (memantine hydrochloride), adamantan-1-amine (amantadine), 2-(2-chlorophenyl)-2-(methylamino)cyclohexan-1-one (ketamine), 1-(1-phenylcyclohexyl)piperidine (phencyclidine), argiotoxins, such as (2S)-N-[5-[3-[3-[[(2S)-2-amino-5-(diaminomethylideneamino)pentanoyl]amino]propylamino]propylamino]pentyl]-2-[[2-(2,4-dihydroxyphenyl)acetyl]amino]butanediamide (Argiotoxin-636), (1S,9S,10S)-17-methyl-17-azatetracyclo[7.5.3.01,10.02,7]heptadeca-2(7),3,5-trien-4-ol (dextrorphan), 1-(3-ethylphenyl)-1-methyl-2-naphthalen-1-ylguanidine (aptiganel), polyamines, such as 2-[4-(diaminomethylideneamino)butyl]guanidine (arcaine), *N*'-(2-aminoethyl)ethane-1,2-diamine (diethylenetriamine) and butane-1,4-diamine (putrescine), 1,3,3,5,5-pentamethylcyclohexan-1-amine (neramexane), 2-amino-2-(2-chlorophenyl)cyclohexan-1-one (norketamine) and (1*S*,9*R*)-1-methyl-16-azatetracyclo[7.6.1.0^{2,7}.0^{10,15}]hexadeca-2,4,6,10,12,14-hexaene (dizocilpine or MK801).

In the context of the present invention, the term "treatment and/or prevention" as used herein encompasses preventing, delaying, reducing the risk of developing, ameliorating or curatively treating the neurodegenerative diseases mentioned herein. Treatment may be symptomatic treatment or disease modifying treatment. In some embodiments, the prevention as used herein refers to preventing the neurodegenerative diseases mentioned herein. Prevention as used in relation to prevention of neurodegenerative disease may be the prevention or reduction in progression of disease. An individual already diagnosed with a neurodegenerative disease may upon treatment with the conjugate molecule of the present invention experience a reduced progression of the disease, that is a slower further deterioration, compared to a subject not suffering from the neurodegenerative disease.

In the context of the present invention, the term "subject in need thereof" is a subject, particularly a human, diagnosed with a neurodegenerative disease. The diagnosis of the neurodegenerative disease and choice of suitable treatment course is handled by a general practitioner (GP), possibly in collaboration with a specialist, such as a psychiatrist, an elderly care physician (in particular for age-related neurodegenerative diseases, such as e.g. Alzheimer's disease and dementia), and/or a neurologist. The subject diagnosed with the neurodegenerative disease may, following the initial diagnosis, go for regular check-ups with the GP and/or specialist to track the progression of the neurodegenerative disease.

### Brief description of the figures

The above, as well as additional objects, features, and advantages of the present invention is better understood through the following illustrative and detailed description of embodiments of the present invention, with reference to the appended drawings, wherein:
Fig. 1 shows an example of a peptide and an NMDAR antagonist conjugate,
Fig. 2 shows the mechanism by which the NMDAR antagonist, herein MK801 (dizocilpine), is released from the conjugate of figure 1,

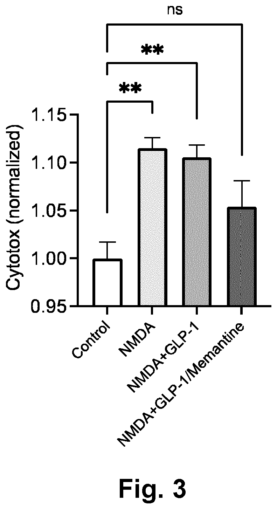

Fig. 3 shows the effect of GLP-1 Pen40/memantine on neuroprotection in primary hippocampal neurons of mice,

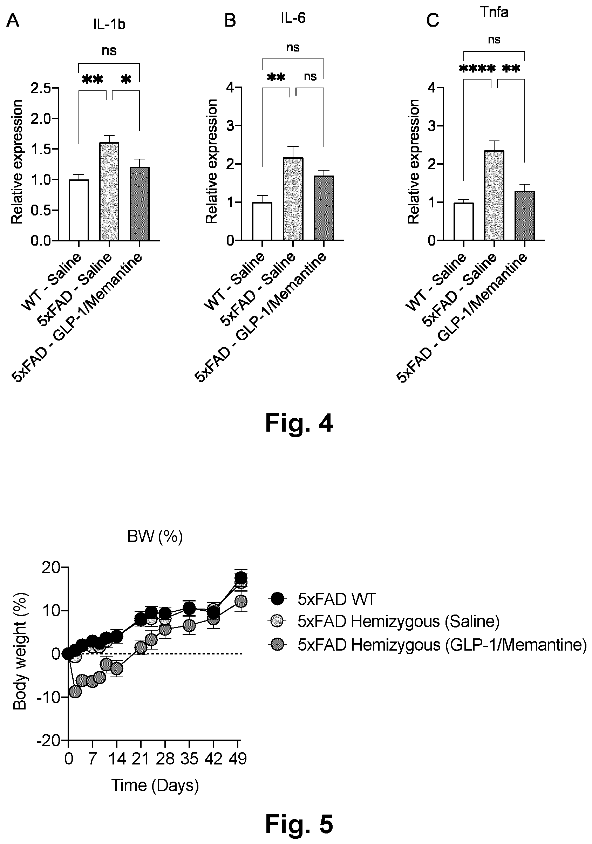

Fig. 4 shows the effect of GLP-1 Pen40/memantine on gene expression of pro-inflammatory markers IL-1b, IL-6 and TNF-α in hippocampus of mice,
Fig. 5 shows the effect of GLP-1 Pen40/memantine on body weight progression in 5xFAD mice,
Fig. 6 shows an amino acid sequence alignment between co-agonist GLP-1/GIP of SEQ ID NO: 5 and the modified GLP-1 peptide of SEQ ID NO: 1, wherein X₁ is D-alanine, D-serine, alpha-aminoisobutyric acid, N-methyl-serine, glycine, or valine, X₂ is cysteine (hCys40/Cys40) or L-penicillamine (Pen40) and Cₑₓ is the Exendin-4 extension (SEQ ID NO:9) at the C-terminal of the peptide,
Fig. 7 shows an amino acid sequence alignment between tri-agonist GLP-1/GIP/GCG of SEQ ID NO: 7 and the modified GLP-1 peptide of SEQ ID NO: 1, wherein X₁ is D-alanine, D-serine, alpha-aminoisobutyric acid, N-methyl-serine, glycine, or valine, X₂ is cysteine (hCys40/Cys40) or L-penicillamine (Pen40), and Cₑₓ is the Exendin-4 extension (SEQ ID NO:9) at the C-terminal of the peptide,
Fig. 8 shows the number of amyloid plaques in frontal cortex (left graph) and hippocampus (right graph) following 49 days of treatment with saline (control) or GLP-1/Memantine in 5xFAD mice.

### Detailed description

Fig. 1 shows an example of a peptide and NMDAR antagonist conjugate 100, which consists of MK801 (Dizocilpine) 101 chemically appended to a C-terminal cysteine 102 of the peptide of SEQ ID NO:1 103 through a chemical linker 104, the chemical linker 104 comprising a disulfide group 105. A side chain 106 of the C-terminal cysteine 102, may optionally be derivatised, such that length n of the side chain 106 is 1 or 2 carbon atoms and/or R is hydrogen or methyl. A modification called hCys40 of the side chain 106 has length n = 2 carbon atom and R = hydrogen. A modification called hCys40 of the side chain 106 has length n = 1 carbon atom and R = methyl. Regular cysteine is called Cys40.

Fig. 2 displays the mechanism by which the NMDAR antagonist, herein MK801 (dizocilpine), is released from the conjugate 100 of figure 1. The chemical linker 104 comprising a disulfide group 105 is self-immolative and may be reduced in a reducing environment (not shown) such as an intracellular environment to produce thiol groups, separating the peptide part of the conjugate 107 from the MK801 part 108 of the conjugate. On the MK801 part 108 of the molecule, a liberated nucleophilic thiol 109 undergoes spontaneous intramolecular cyclization to release MK801 as the native unmodified MK801 drug (free form of MK801).

Fig. 3 shows the effect of GLP-1 Pen40/memantine on neuroprotection in primary hippocampal neurons. Primary rat hippocampal neurons were incubated with either buffer, NMDA (to induce neurotoxicity), NDMA+GLP-1 or NMDA+GLP-1/memantine conjugate for 24 hours. Media was harvested and analysed for Lactate Dehydrogenase (LDH) activity as an indirect measure of cellular cytotoxicity. The data shows that the conjugate of GLP-1/memantine, but not GLP-1 peptide alone, prevents the NMDA-induced neurotoxicity. The data of Fig. 3 further shows that the synergistic effect of GLP-1 and memantine is only obtained when the two compounds are conjugated, as evident from the significant reduction in cytotoxicity only seen for the conjugated molecule of GLP-1/memantine.

Fig. 4 shows the effect of GLP-1 Pen40/memantine on gene expression of pro-inflammatory markers IL-1b, IL-6 and TNF-α in hippocampus following compound treatment for 49 days. Expression (as measured by mRNA) of pro-inflammatory markers, IL-1b, IL-6 and TNF-α increased across the board in the 5xFAD AD mouse model. Notably, treatment with the conjugate of GLP-1/memantine completely abolishes the induction of inflammation in the 5xFAD mouse model.

Fig. 5 shows the effect of GLP-1 Pen40/memantine on body weight progression in 5xFAD mice. Female wild-type (WT) control mice + vehicle treatment (n=12), 5xFAD AD mice + vehicle treatment and 5xFAD AD mice + GLP-1/memantine (100nmol/kg/day) treatment were subcutaneously injected daily for 49 days following which brain regions of the mice were dissected and saved for *ex vivo* analyses. It was shown that treatment with the conjugate of GLP-1/memantine elicits only a transient weight loss, thus no long-term weight loss is observed in this *in vivo* Alzheimer's disease model.

Fig. 8 shows the number of amyloid plaques in frontal cortex (left graph) and hippocampus (right graph) following 49 days of treatment with saline or GLP-1/Memantine in 5xFAD mice. Amyloid plaques are commonly found in subjects suffering from Alzheimer's and is caused by abnormal levels of the naturally occurring protein beta-amyloid 42 which clump together to form plaques that collect between neurons and disrupt cell function. Thus, patients suffering from Alzheimer's display elevated levels of amyloid plaques. It was shown that treatment with the conjugate of the present invention, as exemplified by GLP-1 conjugated with memantine, results in reduced formation of amyloid plaques. Thus, without being bound by any particular theory, the inventors postulate that the conjugated molecule according to the present invention has a neuroprotective role.

Overall, the presented data demonstrate that chemical conjugation of a glucagon superfamily peptide analogue, such as the GLP-1 analogue, and an NMDAR antagonist represents a novel medicinal strategy for treatment of neurodegenerative diseases. Conjugates based on this strategy are superior in providing neuroprotection in primary hippocampal neurons relative to the peptide control and are not flawed with adverse central effects of NMDAR antagonism.

In addition to the experiments and findings reported above, the skilled person will appreciate that other tests may also be carried out to evaluate the effect of the conjugated molecule of the present invention in treatment of neurodegenerative diseases. Such experiments or tests include, but are not limited to, behavioural tests commonly used to assess cognitive deficits in rodents may. Examples of such behavioural tests include, but are not limited to, the Y-maze test to evaluate short-term memory, the step-through passive avoidance test to assay long-term memory, as well as the open-field test, the rotarod performance test, the Morris water maze, novel object recognition test, radial arm maze, fear conditioning and passive avoidance learning.

### Examples

### Example 1: Preparation of peptides and peptide-NMDAR antagonist conjugates.

**Materials:** All solvents and reagents were purchased from commercial sources and used without further purification. H-Rink amide ChemMatrix^{®} resin was used for peptide elongation. Unless otherwise stated Fmoc-protected (9-fluorenylmethyl carbamate) amino acids were purchased from Iris-Biotech or Gyros Protein Technologies, and H-Rink amide ChemMatrix^{®} resin, 35-100 mesh; loading of 0.40 - 0.60 mmol/g from Sigma Aldrich. The commercially available *N^{α}*-Fmoc amino acid building blocks were purchased as the following sidechain protected analogs: Arg, Pmc; Asp, O^{t}Bu; Cys, Trt; Gln, Trt; His, Trt; Lys, Trt; Ser, ^{t}Bu; and Trp, Boc (Pmc = 2,2,5,7,8-pentamethylchoman-6-sulfonyl, O^{t}Bu = *tert*-butyl ester, Trt = trityl, Boc = *tert*-butyloxycarbonyl, and ^{t}Bu = *tert*-butyl ether).

All peptides and conjugates of peptides and NMDAR antagonists were characterized by analytical reverse phase ultra-performance liquid chromatography (RP-UPLC) (Waters) and electrospray ionization liquid chromatography mass spectrometry (ESI-LCMS) coupled to a Agilent 6410 Triple Quadrupole Massfilter with a C18 column (Zorbax Eclipse, XBD-C18, 4.6 × 50 mm). The ESI-LCMS was eluting with a binary buffer system composed of H₂O:MeCN:TFA (A: 95:5:0.1, B: 5:95:0.1) at a flow rate of 0.75 mL/min. Purities were determined by RP-UPLC equipped with a C18 column (Acquity UPLC BEH C18, 1.7 µm, 2.1 × 50 mm) eluting with a binary buffer system composed of H₂O:MeCN:TFA (A: 95:5:0.1, B: 5:95:0.1) at a flow rate of 0.45 mL/min.

**Automated peptide synthesis protocol for Fmoc-protection scheme:** Peptides were prepared as their C-terminally amidated derivatives using a Prelude X, induction heating assisted, peptide synthesizer (Gyros Protein Technologies, Tucson, AZ, USA) with 10 mL glass vessels. All reagents were freshly prepared as stock solutions in DMF: Fmoc-protected amino acid (0.2 M), HCTU (0.5 M), DIPEA (1.0 M) and piperidine (20 % v/v). Peptide elongation was achieved by consecutive synthetic manipulations using the following protocol: Deprotection (2 × 2 min, RT, 300 rpm shaking) and coupling (2 × 5 min, 75°C, 300 rpm shaking, for Arg and His 2 × 5 min, 50°C, 300 rpm shaking). Peptides were prepared using double and triple couplings consisting of AA/HCTU/DIPEA (ratio 1:1.25:2.5) in 5-fold excess compared to the resin.

**Peptide cleavage:** The synthesised peptides were liberated from the peptidyl resin by addition of 1.5 mL cleavage cocktail (2.5% EDT, 2.5% H₂O, 2.5% TIPS, 2.5% thioanisole in TFA) per 100 mg peptidyl resin followed by agitation for 2 hours. The crude peptides were precipitated in cold diethyl ether, centrifuged at 2500 × g for 10 min at 4°C, re-dissolved in MeCN:H₂O:TFA (ratio 1:1:0.01), filtered and lyophilized.

**Purification:** The crude peptide or conjugates of peptides and NMDAR antagonist was analyzed by RP-UPLC and ESI-LCMS or MALDI-TOF mass spectrometry prior to purification. Purifications were performed with a reverse-phase high-performance liquid chromatography (RP-HPLC) system (Waters) equipped with a reverse phase C18 column (Zorbax, 300 SB-C18, 21.2 × 250 mm) and eluting with a linear gradient (flow rate 20 mL/min) using a binary buffer system of H₂O:MeCN:TFA (A: 95:5:0.1; B: 5:95:0.1). Fractions were collected at intervals of 0.3 minutes and characterized ESI-LCMS. Purity was determined by RP-UPLC at 214 nm, and fractions with purities >95% were pooled and lyophilized. The final lyophilized products were used in further experiments.

**Conjugation protocol for assembly of conjugates of peptides and NMDAR antagonists:** The pure peptide and the pure thiopyridyl-activated NMDAR antagonist conjugate was dissolved in a binary solvent system (A: DMF; 6 M Guanidine, 1.5 M Imidazole in H₂O at pH = 8) (ratio 7:1) and agitated for at least 2 hours. The crude reaction mixture was monitored by analytical RP-UPLC and ESI-LCMS. Upon completion, the reaction mixture was diluted with buffer A and buffer B and purified directly using RP-HPLC eluting with a linear gradient.

**Desalting:** All peptides were desalted prior to biological experiments. Desalting was performed by consecutively re-dissolving the peptide or the conjugate of a peptide and an NMDAR antagonist in dilute aqueous 0.01 M HCl followed by lyophilization, repeated 3 times. The purity of the peptide or the conjugate was monitored by RP-UPLC and ESI-LCMS before being used for *in vivo* or *in vitro* experiments.

### Preparation of GLP-1 Cys40/Memantine (Cysteine linked).

A GLP-1 peptide with the amino acid sequence of SEQ ID NO:1 was synthesized using the Fmoc protocol as described above and conjugated to a chemical linker derivatized memantine analog. Synthesis of chemical linker derivatized memantine was performed via the synthetic route shown in fig. 15. The first step in the synthethic route took place in MeOH at room temperature for 2 hours. The second step was carried out in CH₂Cl₂ in the precense of pyridine at 0°C for 2 hours. The third step was caried out in DMF in the presence of *N,N*-Diisopropylethylamine (DIPEA) at 55°C for 5 days. The final step (conjugation) was performed in a 6M guanidine, 1.5M imidazole buffer at room temperature for 2 hours.

**2'-Pyridyldithio ethanol.** In a dry round-bottomed flask equipped with a magnetic stirring bar and under N₂ atmosphere, 2'-aldrithiol (4.71 g, 21.3 mmol, 3 equiv.) was dissolved in dry MeOH (20mL), followed by dropwise addition of 2-mercaptoethanol (0.56 g, 7.1 mmol, 0.5 mL, 1 equiv.) via a syringe. The reaction was left for 2 hours at ambient temperature before concentrated *in vacuo.* The crude yellow oil was purified by silica gel flash chromatography (EtOAc:CH₂Cl₂, 2:8), affording 2'-Pyridyldithio ethanol as a clear oil (1.33 g, 100 %). R*_{f}* = 0.48; ¹H NMR (600 MHz, Chloroform-*d*) δ 8.49 (d, *J* = 5.0 Hz, 1H), 7.57 (td, *J* = 7.7, 1.8 Hz, 1H), 7.44 - 7.36 (m, 1H), 7.16 - 7.11 (m, 1H), 5.32 (s, 1H), 3.88 - 3.73 (m, 2H), 3.01 - 2.89 (m, 2H); ¹³C NMR (151 MHz, CDCl₃) δ 159.31, 149.86, 137.00, 122.12, 121.57, 58.37, 42.83.

**4-nitrophenyl (2-(pyridin-2-yldisulfaneyl)ethyl) carbonate.** To a dry round-bottomed flask equipped with a magnetic stirring bar and under N₂-atmosphere, 2'-Pyridyldithio ethanol (1.33 g, 7.1 mmol, 1 equiv.) and dry pyridine (0.56 g, 8.5 mmol, 0.575 mL, 1.2 equiv.) was diluted in anhydrous CH₂Cl₂ (15 mL). The reaction mixture was cooled to 0 °C and nitrophenyl chloroformate (1.72 g, 8.5 mmol, 1.2 equiv.) was added in one portion. The reaction was stirred for 10 minutes, allowed to reach ambient temperature and left for 2 hours under stirring. The reaction was diluted to 50 mL and extracted with 3x H₂O (30 mL) and brine (30 mL), dried over MgSO₄, filtered and concentrated *in vacuo.* The crude oil was purified by silica gel flash chromatography (Heptanes:EtOAc, 2:1), affording 4-nitrophenyl (2-(pyridin-2-yldisulfaneyl)ethyl) carbonate as a clear viscuous oil (2.21 g, 89 %). R*_{f}* = 0.34; Purity >95 % (HPLC), R*ₜ* = 15.99 min; UPLC/MS (ESI): m/z calcd. for C₁₄H₁₂N₂O₅S₂ [M+H]⁺ = 353.0, found 353.3 m/z; ¹H NMR (600 MHz, DMSO-*d*₆) δ 8.47 (ddd, *J* = 4.8, 1.9, 0.9 Hz, 1H), 8.35 - 8.26 (m, 2H), 7.84 (td, *J* = 7.8, 1.8 Hz, 1H), 7.78 (dt, *J* = 8.1, 1.1 Hz, 1H), 7.58 - 7.48 (m, 2H), 7.26 (ddd, *J* = 7.3, 4.8, 1.1 Hz, 1H), 4.48 (t, *J* = 6.0 Hz, 2H), 3.24 (t, *J* = 6.1 Hz, 2H); ¹³C NMR (151 MHz, DMSO) δ 158.65, 155.17, 151.75, 149.66, 145.18, 137.80, 125.40, 122.53, 121.40, 119.52, 66.54, 36.42.

**2-(pyridin-2-yldisulfaneyl)ethyl (3,5-dimethyladamantan-1-yl)carbamate** In a dry round-bottomed flask equipped with a magnetic stirring bar and under N₂, 4-nitrophenyl (2-(pyridin-2-yldisulfaneyl)ethyl) carbonate (707 mg, 2.00 mmol, 1 equiv.) and Memantine hydrochloride (650 mg, 3.00 mmol, 1.5 equiv.) were dissolved in dry DMF (20 mL) and dry DIPEA (260 mg, 6.00 mmol, 0.35 mL, 3 equiv.) was added via syringe. Memantine was not completely dissolved and upon addition of DIPEA, the reaction turned yellow immediately. The reaction was left for 5 days followed by heating to 80 °C. The reaction was then transferred to a separatory funnel with EtOAc (50 mL) and washed exhaustively with 5x half. Sat brine (50 mL) and brine (50 mL) to remove DMF. The organic layer was subsequently extracted 5x 1 M aqueous NaOH (50 mL) (Until the yellow color of the aqueous layer ceased), dried over MgSO₄, filtered and concentrated *in vacuo.* The crude oil was purified by silica gel flash chromatography eluting with a gradient (Heptanes:EtOAc, 9:1 to 3:1), affording 2-(pyridin-2-yldisulfaneyl)ethyl (3,5-dimethyladamantan-1-yl)carbamate as a glassy viscuous oil (540 mg, 54 %). R*_{f}* = 0.26; Purity >95 % (HPLC), R*ₜ* = 19.36 min; UPLC/MS (ESI): m/z calcd. for C₂₀H₂₈N₂O₂S₂ [M+H]⁺ = 393.2, found 393.4 m/z; ¹H NMR (600 MHz, DMSO-*d*₆) δ 8.46 (ddd, *J* = 4.8, 1.9, 0.9 Hz, 1H), 7.85 - 7.75 (m, 2H), 7.25 (ddd, *J* = 7.2, 4.8, 1.2 Hz, 1H), 6.89 (s, 1H), 4.10 (t, *J* = 6.4 Hz, 2H), 3.05 (t, *J* = 6.3 Hz, 2H), 1.69 - 1.63 (m, 2H), 1.54 - 1.43 (m, 4H), 1.31 - 1.20 (m, 5H), 1.07 (s, 2H), 0.80 (s, 6H); ¹³C NMR (151 MHz, DMSO) δ 159.04, 153.78, 149.55, 137.79, 121.21, 119.23, 60.80, 51.40, 50.18, 47.07, 42.22, 37.46, 31.84, 30.05, 29.46.

GLP-1 Cys40 and GLP-1 Cys40/Memantine was prepared using the protocols described above. RP-UPLC and ESI-LCMS analyses determined the purity to > 95%.

### Preparation of GLP-1 Pen40/Memantine (Penicillamine linked).

Synthesis of chemical linker derivatized memantine was performed using the synthetic route disclosed in fig. 15. GLP-1 Pen40 and memantine were conjugated by the chemical reaction shown in fig. 16, which was carried out in 6M guanidine, 1.5M imidazole buffer at room temperature for 2 hours.

### Preparation of GLP-1 Cys40/MK801 (Cysteine linked).

A peptide with the sequence of SEQ ID NO:1 was synthesized using the Fmoc protocol disclosed above and conjugated with a chemical linker derivatized MK801 analog. Synthesis of chemical linker derivatized MK801 was performed via the second synthetic route disclosed in fig. 17. The chemical reation was performed in DMF in the presence of DIPEA at 55°C for 5 days. Linker derivatized MK801 was conjugated to GLP-1 Cys40 by the chemical reation shown in fig. 18. The reaction was performed in a 6M guanidine, 1.5M imidazole buffer at room temperature for 2 hours.

**2-(pyridin-3-yldisulfaneyl)ethyl 5-methyl-10,11-dihydro-5H-5,10-epiminodibenzo[*a,d]*[7]annulen e-12-carboxylate.** In a flame-dried schlenk round-bottomed flask equipped with a magnetic stirring bar and under N₂ atmosphere, MK801 hydrochloride 191 mg, 0.86 mmol, 1.2 equiv.) was dissolved in dry DMF (10 mL) followed by addition of 4-nitrophenyl (2-(pyridin-2-yldisulfaneyl)ethyl) carbonate (253 mg, 0.72 mmol, 1.0 equiv.). Subsequently, dry DIPEA (375 µL, 2.14 mmol, 3.0 equiv.) was added and the solution turned yellow. The reaction was heated to 55 °C in an oil-bath and stirred for 4 days - until UPLC-MS indicated full consumption of the starting material. The reaction was diluted with EtOAc (50 mL) and washed thoroughly with half sat. brine (5x 60 mL), 0.5 M aq. NaOH (5x 60 mL) and brine. The organic layer was collected, dried over MgSO₄, filtered and concentrated in *vacuo.* Purification by preparative HPLC (eluting with isocratic 60% B, over 17 mL/min) followed by lyophilization afforded **11** as a clear solid (250.2 mg, 80.1 %); Purity >95 % (HPLC), R*ₜ* = 18.17 min; UPLC/MS (ESI): m/z calcd. for C₂₄H₂₂N₂O₂S₂ [M+H]⁺ = 435.1, found 435.4; ¹H NMR (600 MHz, DMSO-*d*₆) δ 8.41 (dt, *J* = 4.8, 1.4 Hz, 1H), 7.68 (dt, *J* = 7.9, 4.1 Hz, 2H), 7.45 (d, *J =* 7.1 Hz, 1H), 7.38 = 7.31 (m, 1H), 7.25 - 7.15 (m, 4H), 7.15 - 7.06 (m, 2H), 7.01 - 6.87 (m, 1H), 5.38 (d, *J* = 5.5 Hz, 1H), 4.27 - 4.13 (m, 2H), 3.59 (dd, *J* = 17.3, 5.7 Hz, 1H), 3.10 (s, 2H), 2.67 - 2.58 (m, 1H), 2.20 (s, 3H); ¹³C NMR (151 MHz, DMSO) δ 158.92, 149.56, 143.37, 139.04, 137.70, 131.78, 130.25, 127.42, 127.34, 127.31, 125.88, 122.12, 121.66, 121.20, 119.19, 65.33, 62.21, 59.20, 37.55.

GLP-1 Cys40/MK801 was prepared from 2-(pyridin-3-yldisulfaneyl)ethyl 5-methyl-10,11-dihydro-5*H*-5,10-epiminodibenzo[*a,d*][7]annulen e-12-carboxylate and GLP-1 Cys40 using the protocol disclosed above. RP-UPLC and ESI-LCMS analyses confirmed the product and determined the purity to >95%.

### Preparation of GLP-1 hCys40/MK801 (Homocysteine linked).

A peptide with the amino acid sequence of SEQ ID NO:1 and the hCys40 modification was synthesized using the Fmoc protocol disclosed above and conjugated with a chemical linker derivatized MK801 analog. The chemical synthesis of linker derivatized MK801 was performed via the synthetic route shown in fig. 19, the chemical reaction being perfomed in 6M guanidine, 1.5M imidazole buffer at room temperature for 2 hours.

GLP-1 hCys40: A peptide with the amino acid sequence of SEQ ID NO:1 and the hCys40 modification was prepared using the protocol disclosed above. RP-UPLC and ESI-LCMS analyses determined the purity to >95%. GLP-1 hCys40/MK801 was prepared using the protocol disclosed above. RP-UPLC and ESI-LCMS analyses determined the purity to >95%.

### Preparation of GLP-1 Pen40/MK801 (Penicillamine linked).

A GLP-1 peptide derivative was synthesized using the Fmoc protocol disclosed above and conjugated with a chemical linker derivatized MK801 analog. The chemical synthesis of the chemical linker derivatized MK801 was performed via the route disclosed in fig. 16.

**GLP-1 Pen40/MK801:** The conjugate was prepared using the protocol disclosed above and by the chemical reaction shown in fig. 20, the chemical reaction being perfomed in 6M guanidine, 1.5M imidazole buffer at room temperature for 2 hours. RP-UPLC and ESI-LCMS analyses determined the purity to >95%.

### Example 2: Neuroprotection in primary hippocampal neurons (Fig. 3)

Primary cultures of hippocampal neurons were prepared from Spra-gue-Dawley rat fetuses (E18) and cells were diluted in Neurobasal medium containing B27 supplement (5x10⁵ cells/mL) in a Poly-L-Lysin pre-coated 24-well cell culture plate. The neurons were kept at 37°C in a humidified incubator with 5% CO₂ atmosphere. After 8 days *in vitro,* cultures were treated with either vehicle, NMDA (100 µM), NMDA+GLP-1 (1 µM) or NMDA+GLP-1/Memantine for 24 hours. Cell culture medium (50 µL) was dispatched to a 96-well ELISA plate and analyzed for Lactate Dehydrogenase (LDH) activity (CytoTox-96, Nonradioactive Cytotoxicity assay, Promega) according to the manufacturer's protocol.

### Example 3: Body weight progression of 5xFAD Alzheimer's disease mouse model (Fig. 5)

Experiments were conducted using female 5xFAD (Jackson Laboratory, 34840-JAX) mice kept on a chow diet (Altromin #1310, Lage, Germany). The pharmacological studies were initiated at 8-week of age and the mice treated with once-daily subcutaneous injections of GLP-1/memantine or saline (control) for 49 days with weekly measurements of body weight. Specifically, compounds were administered at a volume of 5 µL per gram, mice group-housed (n= 2-5 mice per cage) and maintained on a 12-hour light-dark cycle (light 6am-6pm, dark 6pm-6am) at a temperature of 21-23°C. For week 5 and 6, the mice were single-housed and transferred to a digitally ventilated cage system. For week 7, the mice were re-grouped in groups identical to weeks 0 to 4. Finally, mice were euthanized on day 49, tissues harvested, snap-frozen using liquid nitrogen and stored at -80°C prior to analyses.

### Example 4: Gene expression in hippocampus following compound treatment for 49 days.. (Fig. 4)

Hippocampal tissue from Example 3 was homogenized in a Trizol reagent (QIAzol Lysis Reagent, Qiagen) using a stainless steel bead (Qiagen) and a TissueLyser LT (Qiagen) for 3 min at 20 Hz. Then, 200 µl chloroform (Sigma-Aldrich) was added and tubes were shaken vigorously for 15 sec and left at RT for 2 min, followed by centrifugation at 4°C for 15 min at 12,000 x g. The aqueous phase was mixed 1:1 with 70% ethanol and further processed using RNeasy Lipid Mini Kit following the instructions provided by the manufacturer. For muscle tissue, the lysis procedure, descirbed in the enclosed protocol in the Fibrous Tissue Mini Kit (Qiagen), was followed. After RNA extraction, RNA content was measured using a NanoDrop 2000 (Thermo Fisher) and 500 ng of RNA was converted into cDNA by mixing FS buffer and DTT (Thermo Fisher) with Random Primers (Sigma-Aldrich) and incubated for 3 min at 70°C followed by addition of dNTPs, RNase out, Superscript III (Thermo Fisher) and placed in a thermal cycler for 5 min at 25°C, 60 min at 50°C, 15 min at 70°C, and kept at -20°C until further processing.

***qPCR:*** SYBR green qPCR was performed using Precision plus qPCR Mastermix containing SYBR green (Primer Design, #PrecisionPLUS). qPCR was performed in 384-well plates on a Light Cycler 480 Real-Time PCR machine using 2 min preincubation at 95°C followed by 45 cycles of 60 sec at 60°C and melting curves were performed by stepwise increasing the temperature from 60°C to 95°C. Quantification of mRNA expression was performed according to the delta-delta Ct method.

### Example 5: Number of amyloid plaques following 49 days of treatment with saline or GLP-1/Memantine in 5xFAD mice (Fig. 8).

Experiments were conducted using female 5xFAD (Jackson Laboratory, 34840-JAX) mice kept on a chow diet (Altromin #1310, Lage, Germany). The pharmacological studies were initiated at 8-week of age and the mice treated with once-daily subcutaneous injections of GLP-1/memantine or saline (control) for 49 days. Specifically, compounds were administered at a volume of 5 µL per gram, mice group housed (n= 2-5 mice per cage) and maintained on a 12-hour light-dark cycle (light 6am-6pm, dark 6pm-6am) at a temperature of 21-23°C. For week 5 and 6, the mice were single-housed and transferred to a digitally ventilated cage system. For week 7, the mice were re-grouped in groups identical to weeks 0 to 4. On day 49, mice were perfusion-fixated with paraformaldehyde, brains dissected and sliced in 30 micron sections. Every 6th slide through hippocampus (bregma: -2.80 mm to -0.94 mm) and frontal cortex (bregma: 0.14 mm to 2.96 mm) were collected and stained with Thioflavine S to quantify amyloid plaque deposition.

## Claims

1. A conjugated molecule for use in treatment and/or prevention of a neurodegenerative disease in a subject in need thereof, the conjugated molecule comprising a peptide and a N-methyl-D-aspartate receptor (NMDAR) antagonist, wherein the peptide has at least 85% amino acid sequence identity to SEQ ID NO:1 or wherein the peptide is according to SEQ ID NO:1, SEQ ID NO:5, SEQ ID NO:6, SEQ ID NO:7 or SEQ ID NO:8, the peptide being linked to the NMDAR antagonist either directly or through a chemical linker, wherein the NMDAR antagonist is selected from memantine, neramexane, and dizocilpine.

2. The conjugated molecule for use in treatment and/or prevention of a neurodegenerative disease in a subject in need thereof according to claim 1, wherein the peptide is an incretin hormone.

3. The conjugated molecule for use in treatment and/or prevention of a neurodegenerative disease in a subject in need thereof according to claim 1 or 2, wherein the peptide is a glucagon-like peptide 1 (GLP-1), a gastric inhibitory peptide (GIP), a combination thereof, and/or any derivatives or analogues thereof.

4. The conjugated molecule for use in treatment and/or prevention of a neurodegenerative disease in a subject in need thereof according to any one of the preceding claims, wherein the NMDAR antagonist in its free form has a dissociation constant K_{d} with an NMDA receptor in the range of 0.5 nM to 1000 nM.

5. The conjugated molecule for use in treatment and/or prevention of a neurodegenerative disease in a subject in need thereof according to any one of the preceding claims, wherein the NMDAR antagonist is linked to the peptide via a cleavable chemical linker, the cleavable chemical linker being selected from acid-cleavable linkers, enzyme-cleavable linkers, peptide-cleavable linkers, and linkers comprising a disulfide group, preferably wherein the peptide is according to SEQ ID NO:1, wherein the amino acid on position 2 counted from the N-terminal of the peptide is alpha-aminoisobutyric acid, and the NMDAR is memantine.

6. The conjugated molecule for use in treatment and/or prevention of a neurodegenerative disease in a subject in need thereof according to claim 5, wherein the cleavable chemical linker is selected from linkers comprising a disulfide group.

7. The conjugated molecule for use in treatment and/or prevention of a neurodegenerative disease in a subject in need thereof according to any one of the preceding claims, wherein the neurodegenerative disease is selected from dementia, mild cognitive impairment, Alzheimer's disease, Parkinson's disease, stroke, traumatic brain injury, Huntington's disease, schizophrenia and depression.

8. The conjugated molecule for use in treatment and/or prevention of a neurodegenerative disease in a subject in need thereof according to any one of the preceding claims, wherein the conjugated molecule is administered in the form of a pharmaceutical composition, wherein the pharmaceutical composition comprises the conjugated molecule or a pharmaceutically acceptable salt thereof, and a pharmaceutically acceptable carrier.

9. The conjugated molecule according to any one of the preceding claims, wherein the conjugated molecule is administered at least once a week for 12 months or more.

10. A pharmaceutical composition comprising a conjugated molecule according to any one of claims 1 to 9, and a pharmaceutically acceptable carrier.

11. The pharmaceutical composition according to claim 10, wherein the pharmaceutically acceptable carrier is a solid pharmaceutically acceptable carrier selected from excipients, diluents, solubilisers, lubricants, suspending agents, binders, preservatives, wetting agents, tablet disintegrating agents and an encapsulating material.

12. The pharmaceutical composition according to any one of claims 10 or 11, wherein the pharmaceutical composition is in the form of a powder, a tablet, a pill, a capsule, a cachet, a suppository, and a dispersible granule.

13. The pharmaceutical composition according to any one of claims 10 to 12, wherein the pharmaceutical composition is configured for subcutaneous administration, intramuscular administration, intraperitoneal administration, intravenous administration or oral administration.

14. The pharmaceutical composition according to any one of claims 10 to 13, wherein the pharmaceutical composition is provided in a unit dose form in an ampoule, a pre-filled syringe, a small volume infusion or in a multi-dose container.

15. The pharmaceutical composition according to any one of claims 10 to 14, wherein the pharmaceutical composition is selected from a suspension, a solution, an emulsion in an oily vehicle, and an emulsion in an aqueous vehicle.

## Patentansprüche

1. Konjugiertes Molekül zur Verwendung bei der Behandlung und/oder Vorbeugung einer neurodegenerativen Erkrankung bei einem Individuum, das dies benötigt, wobei das konjugierte Molekül ein Peptid und einen N-Methyl-D-aspartat-Rezeptor(NMDAR)-Antagonisten umfasst, wobei das Peptid eine Aminosäuresequenzidentität von mindestens 85 % mit SEQ ID NO:1 aufweist oder wobei es sich bei dem Peptid um eines gemäß SEQ ID NO:1, SEQ ID NO:5, SEQ ID NO:6, SEQ ID NO:7 oder SEQ ID NO:8 handelt, wobei das Peptid entweder direkt oder über einen chemischen Linker mit dem NMDAR-Antagonisten verknüpft ist, wobei der NMDAR-Antagonist aus Memantin, Neramexan und Dizocilpin ausgewählt ist.

2. Konjugiertes Molekül zur Verwendung bei der Behandlung und/oder Vorbeugung einer neurodegenerativen Erkrankung bei einem Individuum, das dies benötigt, nach Anspruch 1, wobei es sich bei dem Peptid um ein Inkretinhormon handelt.

3. Konjugiertes Molekül zur Verwendung bei der Behandlung und/oder Vorbeugung einer neurodegenerativen Erkrankung bei einem Individuum, das dies benötigt, nach Anspruch 1 oder 2, wobei es sich bei dem Peptid um ein Glucagon-like Peptide 1 (GLP-1), ein Gastric Inhibitory Peptide (GIP), eine Kombination davon und/oder jegliche Derivate oder Analoge davon handelt.

4. Konjugiertes Molekül zur Verwendung bei der Behandlung und/oder Vorbeugung einer neurodegenerativen Erkrankung bei einem Individuum, das dies benötigt, nach einem der vorhergehenden Ansprüche, wobei der NMDAR-Antagonist in seiner freien Form eine Dissoziationskonstante K_{d} mit einem NMDA-Rezeptor im Bereich von 0,5 nM bis 1000 nM aufweist.

5. Konjugiertes Molekül zur Verwendung bei der Behandlung und/oder Vorbeugung einer neurodegenerativen Erkrankung bei einem Individuum, das dies benötigt, nach einem der vorhergehenden Ansprüche, wobei der NMDAR-Antagonist über einen spaltbaren chemischen Linker mit dem Peptid verknüpft ist, wobei der spaltbare chemische Linker aus säurespaltbaren Linkern, enzymspaltbaren Linkern, peptidspaltbaren Linkern und eine Disulfidgruppe umfassenden Linkern ausgewählt ist, vorzugsweise wobei es sich bei dem Peptid um eines gemäß SEQ ID NO:1 handelt, wobei es sich bei der Aminosäure an Position 2, vom N-Terminus des Peptids gezählt, um Alpha-Aminoisobuttersäure und bei dem NMDAR um Memantin handelt.

6. Konjugiertes Molekül zur Verwendung bei der Behandlung und/oder Vorbeugung einer neurodegenerativen Erkrankung bei einem Individuum, das dies benötigt, nach Anspruch 5, wobei der spaltbare chemische Linker aus eine Disulfidgruppe umfassenden Linkern ausgewählt ist.

7. Konjugiertes Molekül zur Verwendung bei der Behandlung und/oder Vorbeugung einer neurodegenerativen Erkrankung bei einem Individuum, das dies benötigt, nach einem der vorhergehenden Ansprüche, wobei die neurodegenerative Erkrankung aus Demenz, leichter kognitiver Beeinträchtigung, Morbus Alzheimer, Morbus Parkinson, Schlaganfall, Hirntrauma, Chorea Huntington, Schizophrenie und Depression ausgewählt ist.

8. Konjugiertes Molekül zur Verwendung bei der Behandlung und/oder Vorbeugung einer neurodegenerativen Erkrankung bei einem Individuum, das dies benötigt, nach einem der vorhergehenden Ansprüche, wobei das konjugierte Molekül in Form einer pharmazeutischen Zusammensetzung verabreicht wird, wobei die pharmazeutische Zusammensetzung das konjugierte Molekül oder ein pharmazeutisch unbedenkliches Salz davon und einen pharmazeutisch unbedenklichen Träger umfasst.

9. Konjugiertes Molekül nach einem der vorhergehenden Ansprüche, wobei das konjugierte Molekül 12 Monate oder länger mindestens einmal pro Woche verabreicht wird.

10. Pharmazeutische Zusammensetzung, umfassend ein konjugiertes Molekül nach einem der Ansprüche 1 bis 9 und einen pharmazeutisch unbedenklichen Träger.

11. Pharmazeutische Zusammensetzung nach Anspruch 10, wobei es sich bei dem pharmazeutisch unbedenklichen Träger um einen festen pharmazeutisch unbedenklichen Träger handelt, der aus Hilfsstoffen, Verdünnungsmitteln, Lösungsvermittlern, Gleitmitteln, Suspendiermitteln, Bindemitteln, Konservierungsmitteln, Netzmitteln, Sprengmitteln für Tabletten und einem Verkapselungsmaterial ausgewählt ist.

12. Pharmazeutische Zusammensetzung nach Anspruch 10 oder Anspruch 11, wobei die pharmazeutische Zusammensetzung in Form eines Pulvers, einer Tablette, einer Pille, einer Kapsel, eines Cachets, eines Zäpfchens und eines dispergierbaren Granulats vorliegt.

13. Pharmazeutische Zusammensetzung nach einem der Ansprüche 10 bis 12, wobei die pharmazeutische Zusammensetzung für eine subkutane Verabreichung, intramuskuläre Verabreichung, intraperitoneale Verabreichung, intravenöse Verabreichung oder orale Verabreichung konfiguriert ist.

14. Pharmazeutische Zusammensetzung nach einem der Ansprüche 10 bis 13, wobei die pharmazeutische Zusammensetzung in einer Einheitsdosisform in einer Ampulle, einer Fertigspritze, einer Infusion mit geringem Volumen oder in einem Mehrfachdosisbehälter bereitgestellt wird.

15. Pharmazeutische Zusammensetzung nach einem der Ansprüche 10 bis 14, wobei die pharmazeutische Zusammensetzung aus einer Suspension, einer Lösung, einer Emulsion in einem öligen Vehikel und einer Emulsion in einem wässrigen Vehikel ausgewählt ist.

## Revendications

1. Molécule conjuguée pour une utilisation dans le traitement et/ou la prévention d'une maladie neurodégénérative chez un sujet en ayant besoin, la molécule conjuguée comprenant un peptide et un antagoniste du récepteur N-méthyl-D-aspartate (NMDAR), dans laquelle le peptide possède au moins 85 % d'identité de séquence d'acides aminés avec SEQ ID NO:1 ou dans laquelle le peptide est selon SEQ ID NO:1, SEQ ID NO:5, SEQ ID NO:6, SEQ ID NO:7 ou SEQ ID NO:8, le peptide étant lié à l'antagoniste NMDAR soit directement, soit par l'intermédiaire d'un lieur chimique, l'antagoniste NMDAR étant choisi parmi la mémantine, le néramexane et la dizocilpine.

2. Molécule conjuguée pour une utilisation dans le traitement et/ou la prévention d'une maladie neurodégénérative chez un sujet en ayant besoin selon la revendication 1, dans laquelle le peptide est une hormone incrétine.

3. Molécule conjuguée pour une utilisation dans le traitement et/ou la prévention d'une maladie neurodégénérative chez un sujet en ayant besoin selon la revendication 1 ou 2, dans laquelle le peptide est un peptide 1 analogue au glucagon (GLP-1), un peptide inhibiteur gastrique (GIP), une combinaison de ceux-ci et/ou tout dérivé ou analogue de ceux-ci.

4. Molécule conjuguée pour une utilisation dans le traitement et/ou la prévention d'une maladie neurodégénérative chez un sujet en ayant besoin selon l'une quelconque des revendications précédentes, dans laquelle l'antagoniste NMDAR sous sa forme libre a une constante de dissociation K_{d} avec un récepteur NMDA dans la plage de 0,5 nm à 1 000 nM.

5. Molécule conjuguée pour une utilisation dans le traitement et/ou la prévention d'une maladie neurodégénérative chez un sujet en ayant besoin selon l'une quelconque des revendications précédentes, dans laquelle l'antagoniste NMDAR est lié au peptide par l'intermédiaire d'un lieur chimique clivable, le lieur chimique clivable étant choisi parmi des lieurs clivables par acide, des lieurs clivables par une enzyme, des lieurs clivables par un peptide, et des lieurs comprenant un groupe disulfure, de préférence dans laquelle le peptide est selon SEQ ID NO:1, dans laquelle l'acide aminé en position 2 compté à partir de l'extrémité N-terminale du peptide est l'acide alpha-aminoisobutyrique, et le NMDAR est la mémantine.

6. Molécule conjuguée pour une utilisation dans le traitement et/ou la prévention d'une maladie neurodégénérative chez un sujet en ayant besoin selon la revendication 5, dans laquelle le lieur chimique clivable est choisi parmi les lieurs comprenant un groupe disulfure.

7. Molécule conjuguée pour une utilisation dans le traitement et/ou la prévention d'une maladie neurodégénérative chez un sujet en ayant besoin selon l'une quelconque des revendications précédentes, dans laquelle la maladie neurodégénérative est sélectionnée parmi la démence, une déficience cognitive légère, la maladie d'Alzheimer, la maladie de Parkinson, un accident vasculaire cérébral, un traumatisme crânien, la maladie de Huntington, la schizophrénie et la dépression.

8. Molécule conjuguée pour une utilisation dans le traitement et/ou la prévention d'une maladie neurodégénérative chez un sujet en ayant besoin selon l'une quelconque des revendications précédentes, dans laquelle la molécule conjuguée est administrée sous la forme d'une composition pharmaceutique, dans laquelle la composition pharmaceutique comprend la molécule conjuguée ou un sel pharmaceutiquement acceptable de celle-ci, et un support pharmaceutiquement acceptable.

9. Molécule conjuguée selon l'une quelconque des revendications précédentes, dans laquelle la molécule conjuguée est administrée au moins une fois par semaine pendant 12 mois ou plus.

10. Composition pharmaceutique comprenant une molécule conjuguée selon l'une quelconque des revendications 1 à 9, et un support pharmaceutiquement acceptable.

11. Composition pharmaceutique selon la revendication 10, dans laquelle le support pharmaceutiquement acceptable est un support pharmaceutiquement acceptable solide choisi parmi les excipients, les diluants, les solubilisants, les lubrifiants, les agents de suspension, les liants, les conservateurs, les agents mouillants, les agents de désintégration de comprimé et un matériau d'encapsulation.

12. Composition pharmaceutique selon l'une quelconque des revendications 10 ou 11, la composition pharmaceutique étant sous la forme d'une poudre, d'un comprimé, d'une pilule, d'une capsule, d'un cachet, d'un suppositoire et d'une granule dispersible.

13. Composition pharmaceutique selon l'une quelconque des revendications 10 à 12, dans laquelle la composition pharmaceutique est configurée pour une administration sous-cutanée, une administration intramusculaire, une administration intrapéritonéale, une administration intraveineuse ou une administration orale.

14. Composition pharmaceutique selon l'une quelconque des revendications 10 à 13, la composition pharmaceutique étant fournie sous une forme posologique unitaire dans une ampoule, une seringue préremplie, une perfusion à petit volume ou dans un récipient multidose.

15. Composition pharmaceutique selon l'une quelconque des revendications 10 à 14, dans laquelle la composition pharmaceutique est choisie parmi une suspension, une solution, une émulsion dans un véhicule huileux, et une émulsion dans un véhicule aqueux.
